# EUROPÄISCHE PATENTSCHRIFT

(11) **EP 2 262 553 B1**
(45) Veröffentlichungstag und Bekanntmachung des Hinweises auf die Patenterteilung: **30.04.2014**
(21) Anmeldenummer: 09714251.7
(22) Anmeldetag: 26.02.2009
(51) Int. Cl.: A61M 5/20, A61M 5/315, A61M 5/24

(54) **INJEKTIONSVORRICHTUNG MIT DOPPELFUNKTIONSFEDER**
INJECTION DEVICE WITH DUAL FUNCTION SPRING
DISPOSITIF D'INJECTION AVEC RESSORT À DOUBLE FONCTION

(30) Priorität: 29.02.2008 DE 102008011885
(43) Veröffentlichungstag der Anmeldung: 22.12.2010
(73) Patentinhaber: Tecpharma Licensing AG, 3401 Burgdorf (CH)
(72) Erfinder: EICH, Adrian, CH-3374 Wangenried (CH); HORISBERGER, Aurèle, CH-4123 Allschwil (CH); HOSTETTLER, Patrick, CH-3415 Hasle b. Brugdorf (CH); KLADIWA, Malte, CH-3008 Bern (CH); MEIER, Stefan, CH-3270 Aarberg (CH); STETTLER, Peter, CH-3423 Ersigen (CH); WITTMANN, Juergen, CH-3400 Burgdorf (CH)
(86) Internationale Anmeldenummer: PCT/CH2009/000078
(87) Internationale Veröffentlichungsnummer: WO 2009/105909

(56) Entgegenhaltungen:
- EP-A- 0 554 995
- WO-A-00/41754
- WO-A-2004/002557
- US-A1- 2004 236 285

## Beschreibung

Die Erfindung betrifft eine Injektionsvorrichtung zur Verabreichung eines Produkts, insbesondere eines Medikaments. Bei dem Medikament kann es sich z. B. um ein Insulin oder Wachstumshormon oder ein anderes injzierbares Medikament handeln.

Aus dem Stand der Technik ist bekannt, eine ein Produkt enthaltende Ampulle an einer Injektionsvorrichtung anzubringen, siehe z.B. EP 0 554 995. Der Oberbegriff des Anspruchs 1 ist auf diesem Dokument basiert.

Es ist bekannt, dass die Ampulle in ihrem an der Injektionsvorrichtung befestigten Zustand in einem festen Sitz z.B. in einem Ampullenhalter fixiert ist. Dies kann z.B. durch Klemmung geschehen, indem das in dem Ampullenhalter aufgenommene Produktbehältnis z.B. mittels einer Schraubbewegung zwischen dem distalen Ende des Ampullenhalters und einem Anschlag, der z.B. auf das proximale Ende der Ampulle wirkt, eingeklemmt wird.

Es ist eine Aufgabe der vorliegenden Erfindung, eine Injektionsvorrichtung bereit zu stellen, mit der ein Produktbehältnis verlässlich, aber dennoch auf eine einfache und wirtschaftliche Weise an der Injektionsvorrichtung befestigbar ist.

Die Aufgabe wird gelöst durch die Merkmale des Anspruchs 1. Vorteilhafte Weiterentwicklungen ergeben sich aus den abhängigen Ansprüchen.

Die Injektionsvorrichtung zur Ausschüttung eines Produkts kann z.B. so ausgestaltet sein, dass sie eine automatische Produktausschüttung erlaubt. Das Einstechen kann manuell durch den Verwender oder automatisch erfolgen. Zum Beispiel kann der Verwender der Vorrichtung für die automatische Produktausschüttung ein Betätigungselement betätigen, welches ein Antriebsglied, welches z.B. eine Feder freigibt, wobei die Federkraft mittelbar oder unmittelbar auf ein Abtriebsglied wirkt, oder welches den Kolben eines Produktbehältnisses in eine Ausschüttrichtung verschiebt. Das Antriebsglied kann z.B. ein Motor, eine pyrotechnische Ladung oder besonders bevorzugt eine Feder, insbesondere eine mechanische Feder sein. Ist das Antriebsglied ein Energiespeicher, ist es bevorzugt, dass mit dem Betätigungselement der Energiespeicher freigegeben werden kann, so dass die Antriebsbewegung in eine Abtriebsbewegung umsetzbar ist. Zum Beispiel kann die Antriebsbewegung eine Drehbewegung sein, wobei die Abtriebsbewegung eine Linearbewegung sein kann.

Zum Beispiel kann das Antriebselement eine Drehfeder, wie zum Beispiel eine Spiral- oder Uhrenfeder sein, welche bevorzugt um die Längsachse der Injektionsvorrichtung gewunden ist. Die Feder kann sich zum Beispiel mit einem Ende zumindest in Drehrichtung gehäusefest abstützen und mit dem anderen Ende mit dem Abtriebsglied oder einem mit dem Abtriebsglied gekoppelten oder koppelbaren Teil verbunden sein. Dadurch kann vorteilhaft die in der Feder gespeicherte Drehenergie in eine Antriebsbewegung umgesetzt werden. Als besonders vorteilhaft hat sich eine bandförmige Spiralfeder herausgestellt.

Die Injektionsvorrichtung kann zum Beispiel als Einweginjektionsvorrichtung ausgestaltet sein, in der ein werkseitig eingesetztes Produktbehältnis in der Regel nicht austauschbar ist, so dass nach dem Entleeren oder einer Verwendung der Injektionsvorrichtung diese als Ganzes einschließlich Produktbehältnis entsorgt wird. Es kann jedoch von Vorteil sein, die Injektionsvorrichtung als mehrfach verwendbar auszugestalten. Dies ist insbesondere dann sinnvoll, wenn die Injektionsvorrichtung mit hochwertigen Komponenten, die zum Beispiel eine Produktausschüttung komfortabel gestallten und für eine einmalige Verwendung zu teuer sind, ausgestattet ist. Als vorteilhaft hat sich herausgestellt die Antriebsmechanik, zu einer Antriebseinheit zusammenzufassen, wobei das Produktbehältnis an die Antriebseinheit befestigbar und von der Antriebseinheit lösbar ist. Ein leeres Produktbehältnis kann von der Antriebseinheit gelöst, entsorgt und durch ein neues ersetzt werden. Das Produktbehältnis sitzt bevorzugt in einer Produktbehältnisaufnahme die auch als Produktbehältnishalter bezeichnet werden kann. Der Produktbehältnishalter ist bevorzugt hülsenförmig und weist eine Öffnung auf, durch welche das Produktbehältnis in das Innere der Hülse einsetzbar ist. Die Öffnung kann seitlich oder am proximalen Ende des Produktbehältnishalters gebildet sein. Der Produktbehältnishalter kann seitlich einen Bereich aufweisen, durch den von außen in das Innere des Produktbehältnishalters geblickt werden kann, um zum einen erkennen zu können, ob ein Produktbehältnis eingelegt ist und zum anderen um den Füllstand des Produktbehältnisses kontrollieren zu können. Die Produktbehältnisaufnahme kann an ihrem distalen Ende einen Kragen aufweisen, gegen den das Produktbehältnis in seinem eingesetzten Zustand anschlägt. Das Produktbehältnis ist dann in einem festen Sitz mit dem Produktbehältnishalter. Der Produktbehältnishalter eine zum Beispiel konische Form aufweisen, in die das Produktbehältnis für einen festen Sitz geschoben werden kann.

Die Injektionsvorrichtung, insbesondere die Antriebseinheit kann ein Federelement, wie zum Beispiel eine mechanische Feder, eine Gasdruckfeder oder ein anderes Elastizitätsmittel aufweisen, welches auf das Produktbehältnis wirkt oder drückt, insbesondere auf den Teil des Produktbehältnisses relativ zu dem der Kolben verschiebbar ist. Bevorzugt kann das Federelement das Produktbehältnis in den festen Sitz mit dem Produktbehältnishalter drücken. Das Federelement kann insbesondere in Längsrichtung der Injektionsvorrichtung wirken. Das Produktbehältnis kann zum Beispiel als Ampulle oder Kapulle ausgestaltet sein und an seinem proximalen Ende offen und an seinem distalen Ende geschlossen sein. Durch das proximale Ende kann zum Beispiel ein Abtriebsglied der Antriebseinheit sich in das Produktbehältnis erstrecken, um auf den relativ zu der Produktbehältniswand verschiebbaren Kolben zu wirken. Das distale Ende kann zum Beispiel eine Nadel aufweisen oder so ausgestaltet sein, dass eine Nadel angebracht werden kann, die eine fluidische Verbindung zum Innern des Produktbehältnisses herstellt, indem es zum Beispiel ein an dem distalen Ende des Produktbehältnisses angeordnetes Septum durchsticht. Die Wandung des Produktbehältnisses ist bevorzugt zylindrisch. Die Produktionsbehältniswand kann seitlich von der hülsenförmigen Produktbehältnisaufnahme fest oder mit geringem Spiel eingefasst werden.

Die Injektionsvorrichtung kann ein Element aufweisen, welches nach Beendigung oder beim Beenden des Ausschüttvorgangs in eine Ausgangsposition zurückbewegt wird. Dies kann zum Beispiel ein Betätigungselement sein, welches für eine Betätigung bewegbar, insbesondere axial bewegbar, bevorzugt entlang der Längsachse der Injektionsvorrichtung ist. Das Element kann zum Beispiel beim Auslösen des Ausschüttvorgangs in eine Ausschüttposition bewegbar sein, wobei die hierzu erforderliche Bewegungsrichtung der Bewegungsrichtung, die das Element beim Beenden des Ausschüttvorgangs ausführen kann, entgegengesetzt ist. Das Betätigungselement kann zum Beispiel von einem Verwender der Vorrichtung für eine Auslösung der Produktausschüttung betätigt werden und zum Stoppen oder nach dem Beendigen der Produktausschüttung losgelassen werden, so dass es sich wieder in seine Ausgangsposition zurückbewegt. Bei dem Element kann es sich auch um ein mit dem Betätigungselement zumindest in eine Richtung vorzugsweise auch in beide Richtungen axial fest gekoppeltes Element handeln. Ferner kann ein nach Beendigung des Ausschüttvorgangs wieder in eine Ausgangsposition zurückbewegtes oder zurückbewegbares Element ein Teil einer Kupplung, insbesondere ein Kupplungsglied sein, welches bewirkt, dass eine Kupplung bei seiner Bewegung eingekuppelt oder ausgekuppelt wird. Zum Beispiel kann ein solches Kupplungsglied zumindest zeitweise und zumindest in eine Richtung mit dem Betätigungselement gekoppelt sein. Zum Beispiel kann das Kupplungsglied das Betätigungselement in eine Richtung verschieben, während das Betätigungselement das Kupplungsglied in die andere, entgegengesetzt Richtung, wie zum Beispiel in Richtung distal, verschieben kann.

Das beim Beenden oder Auslösen des Ausschüttvorgangs bewegbare Element kann von dem Federelement, welches auch das Produktbehältnis in seinem festen Sitz drückt, nach Beendigung des Ausschüttvorganges wieder in die Ausgangsposition zurückbewegt werden. Das Federelement ist bevorzugt eine Wendelfeder, welche aus einem drahtförmigen Material gewickelt sein kann. Das Federelement kann parallel, insbesondere konzentrisch zu Längsachse der Injektionsvorrichtung angeordnet sein.

Das Federelement kann eine Doppelfunktion übernehmen, nämlich zum einen das Produktbehältnis in seinen festen Sitz drücken und zum anderen das Element, welches nach Beendigung des Ausschüttvorganges wieder in eine Ausgangsposition zurückbewegt wird, mit der hierfür notwendigen Kraft versorgen. Der Vorteil, das Produktbehältnis mittels einer Feder in den festen Sitz zu drücken, ist der, dass Längentoleranzen des Produktbehältnisses auf einfache Weise ausgeglichen werden können. Ferner kann es auch möglich sein, die Injektionsvorrichtung für Produktbehältnisse verschiedener Hersteller verwendbar zu machen. Ein Vorteil des Federelements mit Doppelfunktion ist, dass separate Federn, wovon eine auf das Produktbehältnis drückt und das andere das Element verschiebt, zu einer Feder zusammengefasst werden können, wodurch eine Kostenersparnis stattfindet.

Das nach der Beendigung des Ausschüttvorgangs wieder in seine Ausgangsposition zurückbewegbare Element kann zum Beispiel eine Bewegung des Abtriebsglieds relativ zu dem Gehäuse sichern, insbesondere sperren, wenn es sich in einem eingekuppelten Zustand befindet, und relativ zum Gehäuse entsichern, insbesondere entsperren, wenn es sich in einem ausgekuppelten Zustand befindet. Bevorzugt ist diese Sicherung eine Drehsicherung. Für einen Ausschüttvorgang wird bevorzugt, dass das Element, das Teil einer Kupplung sein kann, für einen Ausschüttvorgang aus dem Kupplungseingriff bewegt wird. Hierzu kann es zum Beispiel von den Betätigungselement aus dem Kupplungseingriff bewegt werden, indem das Betätigungselement betätigt, insbesondere gedrückt wird, wodurch das Element gegen die Federkraft des Federelements aus dem Kupplungseingriff verschoben wird. Das Element kann relativ zu einem Gehäuse der Injektionsvorrichtung, insbesondere der Antriebseinheit axial verschiebbar und drehfest sein. Die Ausschüttbewegung des Abtriebsglieds, welches als Kolbenstange ausgestaltet sein kann und an dessen Ende ein frei drehbarer Flansch axial fest angeordnet sein kann, relativ zu dem Gehäuse oder einem Eingriffsglied, welches in das Abtriebsglied eingreift, erfolgen. Zum Beispiel kann das Eingriffsglied so in das Abtriebsglied eingreifen, dass das Abtriebsglied relativ zum Eingriffsglied in und/oder gegen die Ausschüttrichtung axial bewegbar, insbesondere schraubbar ist. Zum Beispiel kann das Eingriffsglied ein Innengewinde aufweisen, welches in ein Außengewinde des Abtriebsglieds eingreift. Alternativ könnte das Abtriebsglied von dem Eingriffsglied längsgeführt werden. Das Eingriffsglied kann relativ zu dem Gehäuse drehfest und, aber nicht notwendigerweise, axial fest sein. Bevorzugt kann das Eingriffsglied relativ zu dem Gehäuse axial verschiebbar angeordnet sein, wobei es vorzugsweise bei einem eingesetzten und an der Antriebseinheit befestigten Produktbehältnis axial fest zum Gehäuse ist.

In bestimmten Ausführungen kann das Abtriebsglied, welches für eine Produktausschüttung auf das ausschüttende Produkt, insbesondere über den Kolben wirkt, so mit dem rücksetzbaren Element gekoppelt sein, dass seine Ausschüttbewegung in einem unbefestigten Zustand des Produktbehältnisses gesperrt ist. Somit kann auch erreicht werden, dass bei einem nicht eingesetzten Produktbehältnis ein Auslösen nicht möglich ist. Bevorzugt wird es jedoch, dass das Abtriebsglied bei einem nicht eingesetzten Produktbehältnis für eine Drehbewegung entsperrt ist, so dass bei einem nicht eingesetzten Produktbehältnis oder einer abgenommenen Produktbehältnisaufnahme das Abtriebsglied vom Verwender durch eine Drehbewegung des Abtriebsglieds in die Antriebseinheit, insbesondere in Richtung proximal zurückschraubbar ist. Bevorzugt weist das Gewinde, mit dem das Eingriffsglied in das Abtriebsglied eingreift so eine Steigung auf, dass bei einer axialen Belastung keine Selbsthemmung des Gewindes eintritt. Durch Drücken des Abtriebsglieds, insbesondere auf den Flansch, in Richtung proximal kann dieses auf einfache Weise in Richtung proximal verschoben werden, ohne dass der Verwender eine Drehbewegung auf das Abtriebsglied ausüben muss. Das Eingriffsglied kann bei einem entfernten Produktbehältnis oder einem entfernten Produktbehältnishalter zwischen einer ersten Position und einer zweiten Position bewegbar sein, wobei in der ersten Position das Abtriebsglied von dem Element entkoppelt ist, so dass das Abtriebsglied in oder gegen eine Ausschüttrichtung bewegbar ist. Sofern das Produktbehältnis oder der Produktbehältnishalter an der Antriebseinheit befestigt ist, kann das Eingriffsglied in seine zweite Position verschoben sein, so dass eine Drehbewegung des Abtriebsglieds nicht möglich ist. Zum Beispiel kann das Eingriffsglied durch das Produktbehältnis oder durch die Produktbehältnisaufnahme unmittelbar oder mittelbar, zum Beispiel mittels der Befestigungseinrichtung, verschoben werden. Zum Beispiel kann das Produktbehältnis oder der Produktbehältnishalter einen Teil der Befestigungseinrichtung bilden oder aufweisen. Bei einer Bewegung aus einem unbefestigten Zustand in den befestigten Zustand, insbesondere einer Drehbewegung, vorzugsweise einer kombinierten Dreh-Axialbewegung des Produktbehältnisses oder des Produktbehältnishalters, kann eine Bewegung des Eingriffsglieds erzeugt werden. Allgemein bevorzugt wird, dass das Eingriffsglied so mit der Befestigungseinrichtung gekoppelt ist, dass es beim Befestigen oder Lösen des Produktbehältnisses oder des Produktbehältnishalters an oder von der Antriebseinrichtung axial bewegt wird. Das Eingriffsglied kann zum Beispiel axial fest mit einem Kupplungsglied, wie zum Beispiel einer Kupplungshülse, verbunden sein, das wiederum drehbar zu dem Eingriffsglied sein kann. Das Kupplungsglied kann zum Beispiel hülsenförmig sein und das Abtriebsglied umgeben, oder zumindest so in einem Eingriff mit dem Abtriebsglied sein, dass das Abtriebsglied relativ zu dem Kupplungsglied drehfest aber axial bewegbar ist. Das Kupplungsglied kann beispielsweise in eine Längsnut des Abtriebsglieds eingreifen. Das Kupplungsglied kann Teil eines Übertragungselements sein, welches das Drehmoment des Antriebsglieds auf das Abtriebsglied überträgt. Zum Beispiel kann ein weiteres, mit dem Antriebsglied drehmomentfest verbundenes Element, wie zum Beispiel eine Antriebswelle vorgesehen sein, das mit dem Abtriebsglied gekoppelt oder koppelbar ist, insbesondere indem die Antriebswelle über eine vorzugsweise axial ein- und ausrückbare Kupplung mit dem Abtriebsglied koppelbar ist. Hierzu können das Kupplungsglied und die Antriebswelle Teile der Kupplung bilden, welche ein- und auskuppelbar ist.

Ferner kann das Kupplungsglied Abragungen aufweisen, die mit dem rücksetzbaren Element je nach gewünschtem Schaltzustand in einem Eingriff oder aus einem Eingriff bewegt sind. Bevorzugt ist das mit der Doppelfunktion betraute Federelement zwischen dem zurücksetzbaren Element und dem Produktbehältnis angeordnet. Vorteilhaft wird das rücksetzbare Element von der Feder mit einer in proximale Richtung wirkenden Kraft beaufschlagt, wobei das Produktbehältnis von der Feder mit einer in distale Richtung wirkenden Kraft beaufschlagt wird. Das Federelement kann unmittelbar oder mittelbar auf das Produktbehältnis wirken. Bevorzugt wirkt das Federelement über ein oder mehrere zwischen dem Produktbehältnis und dem Federglied angeordnete Teile auf das Produktbehältnis. Zwischen Produktbehältnis und Federelement kann sich zum Beispiel ein Halteglied befinden, welches mit seinem distalen Ende auf das proximale Ende des Produktbehältnisses drückt. Das Halteglied kann zum Beispiel einen Axialanschlag aufweisen, der beim Entfernen oder bei einem entfernten Produktbehältnis ein vollständiges Entspannen des Federelements verhindert. Zum Beispiel kann der Anschlag in einen Anschlag mit dem Eingriffsglied geraten, so dass das Halteglied nur um einen begrenzten Weg axial bewegbar ist, der jedoch ausreichend groß ist, Längentoleranzen des Produktbehältnisses auszugleichen. Das Halteglied kann relativ zum Eingriffsglied und/oder zu dem Gehäuse verschiebbar sein. Es kann vorteilhaft sichergestellt werden, dass auch bei einem eingelegten Produktbehältnis eine Kraft von der Feder auf das zurücksetzbare Element und zum Beispiel auch auf das Betätigungselement wirkt.

Zwischen Federelement und Betätigungselement können ein oder mehrere Teile, d.h. mindestens ein weiteres Teil, insbesondere zusätzlich zu dem rücksetzbaren Element, angeordnet sein, welches von dem Betätigungselement und/oder von dem Federelement zurücksetzbaren Element verschiebbar ist. Zum Beispiel können zwischen dem Betätigungselement und dem zurücksetzbaren Element ein Lager, welches der Lagerung des Kupplungsglieds quer zur Längsachse dient, und/oder eine Gewindehülse, welche zum Beispiel eine Drehbewegung für Zusatzausstattungen der Injektionsvorrichtung, insbesondere eine Dosisanzeige oder einen Dosisstop bei letzter Dosis, nutzbar macht, oder/und eine Bremseinrichtung, welche die Antriebsgeschwindigkeit begrenzen soll, oder/und ein Antriebsglied, welches die notwendige Antriebsenergie für die Produktausschüttung liefert, angeordnet sein. Die genannten Teile können bei der Betätigung des Betätigungselements zum Beispiel in Richtung distal und bei der Rücksetzbewegung des Federelements in Richtung proximal verschoben werden. Das Federelement hat somit auch noch die Aufgabe, dass es axial angrenzende Teile axial zusammenhält. Ferner kann das Federelement dazu dienen, verschiedene Kupplungen einzukuppeln oder auszukuppeln, das heißt die notwendigen Kupplungskräfte zu liefern.

Das Federelement, welchem die Doppelfunktion zukommt, kann ein separates Teil oder ein von einem Element, an welches die Feder angrenzt, gebildetes Teil sein. Bei einer separaten Feder kann diese zum Beispiel aus einem Kunststoff oder vorzugsweise aus einem Metall sein. Ist das Federelement einteilig mit einem anderen Teil gebildet, kann das Federelement vorzugsweise aus einem Kunststoff sein, da vorteilhaft das Federelement mit dem anderen Teil spritzgegossen sein kann. Somit kann sich der Vorteil ergeben, dass ein teueres Zukaufteil eingespart werden kann. Andererseits kann es erforderlich sein, dass die Feder aus einem Metall sein sollte, wobei dann das Federelement zum Teil von dem Element, mit dem es integral gebildet ist, umgossen sein kann, was auf einfache Weise beim Spritzgießen erfolgen kann. Zum Beispiel kann das Federelement einteilig mit dem zurücksetzbaren Element oder einteilig mit einem Stützring, welcher grundsätzlich als separates Teil zwischen Feder und Haltelement angeordnet sein kann, oder einteilig mit dem Halteelement, welches auf das Produktbehältnis drückt und/oder einen Axialanschlag aufweist, gebildet sein. Es ist auch möglich, Halteelement, Federelement und rücksetzbares Element einteilig auszugestalten.

Die Erfindung wurde anhand mehrerer Ausführungsformen beschrieben. Im Folgenden wird eine Ausführung der Erfindung anhand von Figuren beschrieben. Die hierbei offenbarten Merkmale bilden je einzeln und in Kombination den Gegenstand der Erfindung vorteilhaft weiter. Es zeigen:
- Figur 1: eine Querschnittsansicht eines proximalen Teils einer Injektionsvorrichtung,
- Figur 2: eine perspektivische Ansicht eines Gehäuseteils mit einer Führungsbahn für einen Bajonettverschluss und einer eingesetzten Bajonetthülse,
- Figur 3: eine perspektivische Ansicht der Bajonetthülse aus Figur 2
- Figur 4: eine perspektivische Ansicht der Bajonetthülse aus Figur 3 mit einem darin eingesetzten Eingriffsglied
- Figur 5: eine perspektivische Ansicht einer Bajonetthülse und einer Produktbehältnisaufnahme, die axial in einen drehmomentfesten Eingriff gebracht sind,
- Figur 6: eine perspektivische Ansicht eines Abtriebsglieds mit Flansch und Federglied,
- Figur 7A und 7B: eine Explosionsansicht und eine perspektivische Ansicht einer Bremseinrichtung gemäß einer ersten Ausführungsform,
- Figur 8: eine perspektivische Explosionsansicht einer Bremseinrichtung gemäß einer zweiten Ausführungsform,
- Figur 9: ein Diagramm mit einem schematischen Verlauf der Bremskraft in Abhängigkeit von der Winkelgeschwindigkeit,
- Figur 10: ein Diagramm zur Erläuterung der Bremswirkung bei einer zeitabhängigen Darstellung,
- Figuren 11A und 11B: eine Explosionsansicht und eine perspektivische Ansicht einer auf dem Prinzip einer Wirbelstrombremse wirkenden Bremseinrichtung gemäß einer dritten Ausführungsform,
- Figur 12: eine perspektivische Ansicht einer auf dem Prinzip einer Fliehkraftbremse wirkenden Bremseinrichtung gemäß einer vierten Ausführungsform,
- Figur 13: eine Explosionsansicht einer auf dem Prinzip einer Fluidbremse wirkenden Bremseinrichtung gemäß einer fünften Ausführungsform und
- Figur 14: eine perspektivische Ansicht eines Bremsengehäuses aus Figur 13.

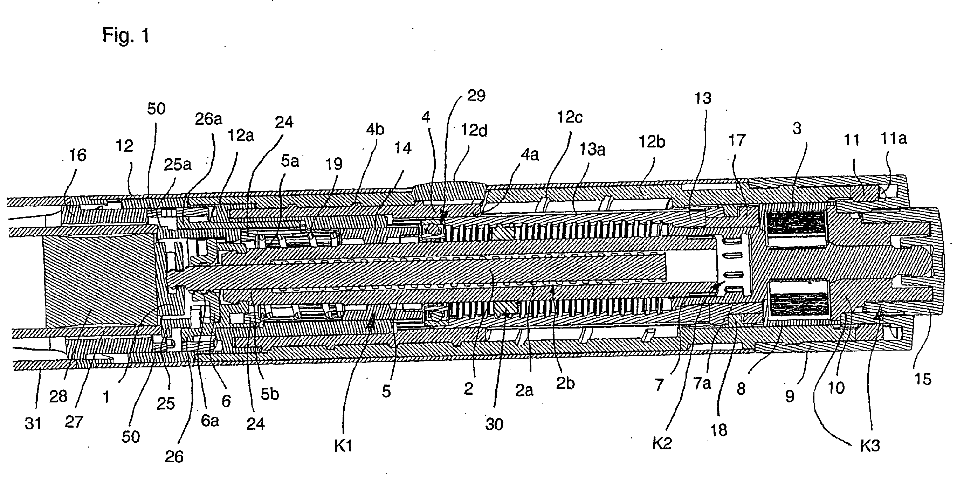

Die in Figur 1 gezeigte Injektionsvorrichtung umfasst eine Antriebseinheit, die bevorzugt mehrfach verwendbar ist, und ein damit verbundenes Produktbehältnis 27, das in einer hülsenformigen z.B. mehrfach verwendbaren Produktbehältnisaufnahme 16 aufgenommen und mit Hilfe der Produktbehältnisaufnahme 16 an der Antriebseinheit befestigbar ist. Das Produktbehältnis 27 kann nach seiner Entleerung von der Injektionsvorrichtung entfernt, entsorgt und gegen ein neues ausgetauscht werden. Das Gehäuse 12 ist wegen der einfacheren Herstellbarkeit und Montierbarkeit mehrteilig gebildet mit damit verbundenen oder eingesetzten Elementen 12a, 12b, wobei das Gehäuse grundsätzlich auch einteilig gebildet sein könnte. Das Produktbehältnis 16 ist mit Hilfe eines Bajonettverschlusses, der von dem Gehäuse 12, der Produktbehältnisaufnahme 16 und der Hülse 50 gebildet wird, an der Antriebseinheit befestigt. Die Produktbehältnisaufnahme 16 wird durch eine Kappe 31 abgedeckt, die an das Gehäuse 12 angesteckt, für eine Verwendung der Injektionsvorrichtung abnehmbar und anschließend wieder aufsteckbar ist.

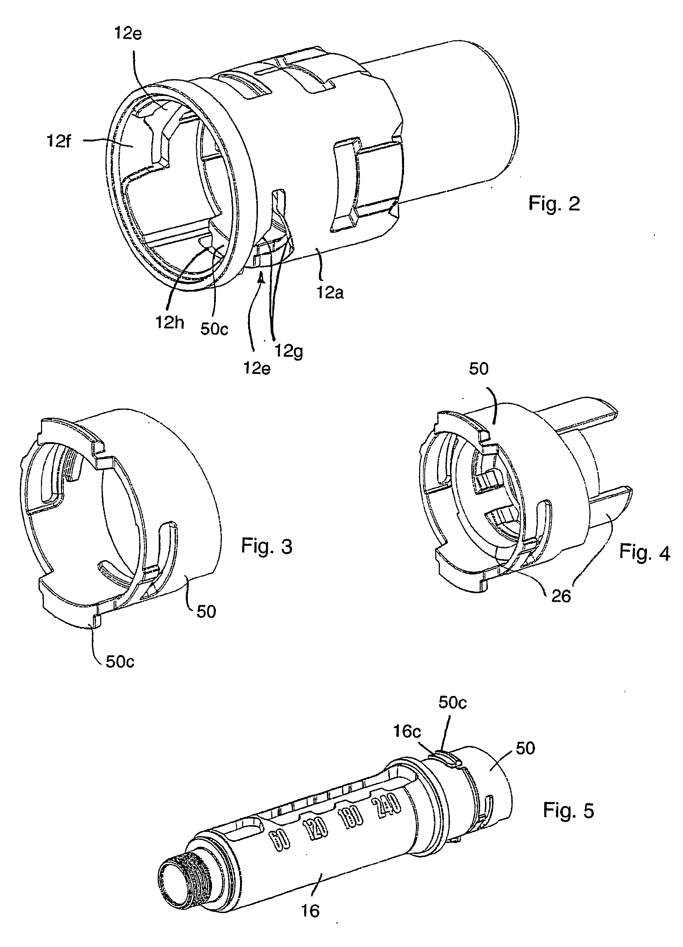

Die Figuren 2 bis 5 zeigen wesentliche Elemente der als Bajonettverschluss ausgestalteten Befestigungseinrichtung. Die Produktbehältnisaufnahme 16 weist einen radial nach außen ragenden Nocken 16c auf und ist an ihrer proximalen Stirnseite so gebildet, dass sie formschlüssig, d.h. drehmomentfest mit der distalen Stirnseite der Hülse 50 verbindbar ist, wie in Figur 5 gezeigt, in der zu Darstellungszwecken das Gehäuseteil 12a weggelassen wurde. Die Hülse 50 weist mindestens einen radial nach außen ragenden Nocken 50c auf, der einen Teil eines Nockens 16c, 50c für die Befestigungseinrichtung bildet. Der Nocken 50c greift in eine in dem Gehäuse 12, insbesondere in dem Gehäuseteil 12a gebildete Führungsbahn 12e, die mindestens eine schräge Fläche 12g aufweist, ein. Bei einer Drehbewegung der Hülse 50 wird die Hülse 50 aufgrund des Eingriffs des Nockens 50c zusätzlich zu der Drehbewegung relativ zu dem Gehäuseteil 12a axial bewegt. Durch die Axialbewegung der Hülse 50 können, wie noch beschrieben wird, verschiedene vorteilhafte Wirkungen erzielt werden.

Zum Anbringen des Produktbehältnisses 27 an die Antriebseinheit wird ein neues Produktbehältnis 27 durch das proximale Ende in die Produktbehältnisaufnahme 16 eingeführt. Anschließend wird die Produktbehältnisaufnahme 16 mit einer axialen Bewegung drehmomentfest an die Hülse 50 angesteckt (Figur 5), wobei die Nocken 16c durch die Öffnung 12f (Figur 2) in die Führungsbahn 12e eingeführt werden. Figur 2 zeigt den Bajonettverschluss ohne die Produktbehältnisaufnahme 16 in einem verriegelten Zustand. In einem entriegelten Zustand, in dem sich die Nocken 50c im Bereich, insbesondere in einer axialen Flucht der Öffnungen 12f befinden, kann die Produktbehältnisaufnahme 16 angesteckt werden. Die Nocken 16c und 50c liegen dann aneinander an und bilden quasi einen gemeinsamen Nocken 16c, 50c (Figur 5). Eine Verdrehung der Produktbehältnishalterung 16 bewirkt eine Mitnahme des Hülse 50. Durch die Schrägen 12g werden die Hülse 50 und die Produktbehältnisaufnahme 16 zusätzlich axial bewegt. Am Ende der Drehung, d.h. beim Erreichen der verriegelten Position befindet sich der gemeinsame Nocken 16c, 50c im Bereich 12h der Führungsbahn 12e, in dem die beiden Nocken 16c und 50c von den Flanken der Führungsbahn 12e axial gegeneinander gespannt werden. Hierzu ist die axiale Breite der Führungsbahn im Bereich 12h in etwa so breit, wie die des gemeinsamen Nockens 16c, 50c.

Wie in Figur 4 gezeigt wird, ist in der Hülse 50, die auch als Bajonetthülse bezeichnet werden kann, eine Führungshülse 26 aufgenommen. Die Führungshülse 26 ist mit dem Gehäuse 12 drehfest und axial bewegbar sowie mit der der Bajonetthülse 50 drehbar und axial fest verbunden. Dies bewirkt, dass bei der Bewegung der Bajonetthülse 50 aus der entriegelten in die verriegelte Position und umgekehrt, die Führungshülse 26 eine längsgeführte Bewegung relativ zum Gehäuse 12 ausführt.

Wie in Figur 1 zu erkennen ist, ist ein Gewindeeinsatz 6 dreh- und axialfest mit der Führungshülse 26 verbunden, insbesondere verrastet. Gewindeeinsatz 6 und Führungshülse 26 können als Eingriffsglied 6, 26 bezeichnet werden. Der Gewindeinsatz 6 weist ein Innengewinde 6a auf, in welchem das Außengewinde 2a eines Abtriebsglieds 2, das in diesem Beispiel auch als Kolbenstange bezeichnet werden kann, geführt wird, so dass, wenn das Abtriebsglied 2 gedreht wird, sich dieses geführt durch das Innengewinde 6a des Gewindeeinsatzes 6 in Abhängigkeit von der Drehrichtung entweder in proximale Richtung oder in distale, d.h. entgegengesetzte Richtung schraubt.

Das Abtriebsglied 2 trägt auf seiner Außenseite ein Gewinde 2a welches von zwei am Umfang gegenüberliegenden, in axiale Richtung verlaufenden Nuten 2b unterbrochen wird. Eine Kupplungshülse 5, die Teil eines Übertragungselements 7, K2, 5 ist, hat an ihrem distalen Ende zwei einander gegenüberliegende radial nach innen gerichtete Abragungen 5a, 5b, welche in die Nuten 2b des Abtriebsglieds 2 ragen. Die Kupplungshülse 5 ist mit dem Eingriffsglied 6, 26 drehbar und axial fest verbunden. Somit ist das Abtriebsglied 2 relativ zu der Kupplungshülse 5 verdrehgesichert und kann relativ zur Kupplungshülse 5 axial bewegt werden, wenn es relativ zu dem Eingriffsglied 2, 26 gedreht wird. Außer während eines Austauschs des Produktbehältnisses 27 ist die Kupplungshülse 5 axial nicht verschiebbar.

Eine an dem proximalen Ende der Injektionsvorrichtung vorgesehene Antriebswelle 7, die Teil des Übertragungselements 7, K2, 5 ist, weist radial nach innen ragende Zähne 7a auf, welche ein Kupplungselement der Kupplung K2 bilden. Durch das Betätigungen, d.h. Drücken eines Betätigungselements 15 in distale Richtung, werden die Antriebswelle 7 und dadurch auch die Zähne 7a in distale Richtung verschoben, wodurch die Zähne 7a in das proximale Ende der Kupplungshülse 5 eingreifen und eine drehmomentfeste, insbesondere formschlüssige Verbindung bilden.

Ein Federelement bzw. eine Antriebsfeder 3, welche in Form einer Spiralfeder oder UhrenFeder ausgebildet sein kann, ist mit einem Ende mit dem Gehäuse 12 über einer Federhülse 8 auf der Außenseite des Federelements 3 verbunden. Die Federhülse 8 ist relativ zu dem Gehäuse 12 verdrehgesichert und axial verschiebbar. Am anderen Ende ist die Antriebsfeder 3 mit der Antriebswelle 7 verbunden. Hierdurch kann eine in dem Federelement 3 gespeicherte Energie als Drehbewegung der Antriebswelle 7 relativ zu dem Gehäuse 12 abgegeben werden. Für eine Produktausschüttung wird die Energie des Federelements 3 über das Übertragungselement 5, K2, 7 in Form einer Drehbewegung an das Abtriebsglied abgegeben, so dass sich dieses relativ zu dem Eingriffsglied 6, 26 in distale Richtung, d.h. in Ausschüttrichtung schraubt und den Kolben 28 verschiebt, der das Produkt aus dem Produktbehältnis 27 ausschüttet.

Zum Einstellen einer zu verabreichenden Produktdosis kann ein Benutzer das als Dosierknopf ausgestaltete Dosierelement 9, das relativ zu dem Gehäuse 12 axialfest ist, drehen. Das Dosierelement 9 ist über die Kupplung K3 mit einem Kupplungsglied 10 verdrehgesichert gekoppelt. Die Kupplung K3 wird gebildet durch Stege oder Nuten oder Zähne des Dosierknopfes 9, welche mit Stegen oder Nuten oder Zähnen der Kupplungsscheibe 10 formschlüssig zusammenwirken, um eine durch Verschieben des Kupplungsglieds 10 in distale Richtung lösbare Kupplung zu bilden. Das Kupplungsglied 10 kann durch Betätigen des Betätigungselements 15 verschoben und somit gelöst werden. In einem unbetätigten Zustand werden die Kupplungen K3 in einem eingekuppelten und die Kupplung K2 in einem ausgekuppelten Zustand gehalten mittels eines Federelements 19, welches die Antriebswelle 7 in proximale Richtung drückt. Während des Dosiseinstellvorgangs ist die Kupplung K3 eingekuppelt, d.h. eine Drehbewegung des Dosierknopfes 9 wird auf das Kupplungsglied 10 übertragen. Das Kupplungsglied 10 ist mit der Antriebswelle 7 axial- und drehfest verbunden und könnte auch einteilig mit der Antriebswelle 7 ausgebildet sein. Die Drehbewegung des Dosierglieds 9 wird aufgrund der ausgekuppelten Kupplung K2 nicht auf die Kupplungshülse 5 übertragen.

Durch Drehung der Antriebswelle 7 wird die mit der Antriebswelle 7 verbundene Antriebsfeder 3 gespannt. Um zu verhindern, dass durch die während des Einstellvorganges gespannte Antriebsfeder 3 der Dosierknopf 9 wieder zurückgedreht wird, ist eine Ratsche 11 oder ein Ratschmechanismus, welcher eine Ratschenfeder 11a z.B. zum Spannen von Halteelementen aufweisen kann, zwischen dem Gehäuse 12 der Injektionsvorrichtung, dessen Bestandteile z.B. ein Mechanikhalter 12a und ein Mechanikhalter 12b sein können, und dem Dosierknopf 9 vorgesehen. Der Ratschenmechanismus kann so ausgestaltet sein, dass nur die Drehung in eine Richtung, insbesondere nur ein Spannen der Antriebsfeder 3 möglich ist. Bevorzugt wird jedoch, dass der Ratschenmechanismus so ausgestaltet ist, dass die Drehung in beide Drehrichtung, insbesondere ein Spannen und Entspannen der Antriebsfeder 3 möglich ist. Durch eine Drehbarkeit in beide Richtungen kann bei der Einstellung der Produktdosis sowohl eine Produktdosis erhöht als auch verringert werden. Die aktuell eingestellte Produktdosis kann über das Fenster 12d von einer Anzeigetrommel 4 abgelesen werden.

Die Drehbewegung der Antriebswelle 7 wird auch auf die Gewindehülse 13 übertragen, welche dreh- und axialfest mit der Antriebswelle 7 verbunden ist und auch einteilig mit dieser ausgebildet sein könnte. Die Gewindehülse 13 trägt auf ihrem äußeren Umfang 13a mindestens eine Nut, in welche mindestens ein Steg 4a der Anzeigetrommel 4 eingreift, so dass eine Drehbewegung der Gewindehülse 13 auf die Anzeigetrommel 4 durch die verdrehsichere Kopplung übertragen wird, wobei eine axiale Relativbewegung zwischen Anzeigetrommel 4 und Gewindehülse 13 möglich ist. Die Anzeigetrommel 4 weist auf ihrer Außenseite ein Gewinde 4b auf, welches in ein Innengewinde 12c des Gehäuseteils 12b eingreift, so dass die Anzeigtrommel 4 durch eine Drehbewegung in axiale Richtung relativ zum Gehäuse 12 vorzugsweise in distale Richtung verschoben wird. Bevorzugt bewegt sich die Anzeigetrommel 4 während eines Einstell- oder Aufdosiervorganges durch Drehung des Dosierknopfes 9 in distale Richtung der Injektionsvorrichtung (in Fig. 1 nach links). Auf der Außenseite der Anzeigetrommel 4 kann eine Markierung, wie z.B. eine Beschriftung, eine Dosisanzeige oder eine Skala vorgesehen sein, welche durch eine Durchbrechung oder ein Fenster 12d im Gehäuse 12b der Injektionsvorrichtung ablesbar ist, wobei sich die Markierung der Anzeigetrommel 4 relativ zum Fenster 12d verschiebt. Die Anzeigetrommel 4 weist an seinem distalen Ende einen in Umfangsrichtung wirkenden Drehanschlag auf, der bei maximaler Dosis in einen Anschlag mit einem entsprechend an dem Gehäuseteil 12a ausgebildeten Gegenanschlag gerät. Der Gegenanschlag wird von einem stirnseitigen Ende eines Ringspalts des Gehäuseteils 12a gebildet. Ein in Umfangsrichtung wirkender Anschlag hat gegenüber einem Axialanschlag den Vorteil, dass geringere Kräfte auf den Anschlag wirken. Die Anzeigetrommel 4 weist ferner an seinem proximalen Ende einen weiteren in Umfangsrichtung wirkenden Drehanschlag auf, der bei minimaler Dosis in einen Anschlag mit einem entsprechend an dem Gehäuse 12b ausgebildeten Gegenanschlag gerät. Der Gegenanschlag wird von dem proximalen Ende des Gewindegangs 12c gebildet.

Nach dem Einstellen der Dosis und Aufziehen der Antriebsfeder 3 durch Drehen des Dosierknopfes 9 ist der Einstellvorgang beendet, wobei bevorzugt ist, dass das Spannen der Feder 3 beim Aufdosieren erfolgt. Zur Dosiskorrektur kann der Dosierknopf 9 einfach in Gegenrichtung gedreht werden, um eine eventuell zu groß eingestellte Dosis wieder zu verkleinern.

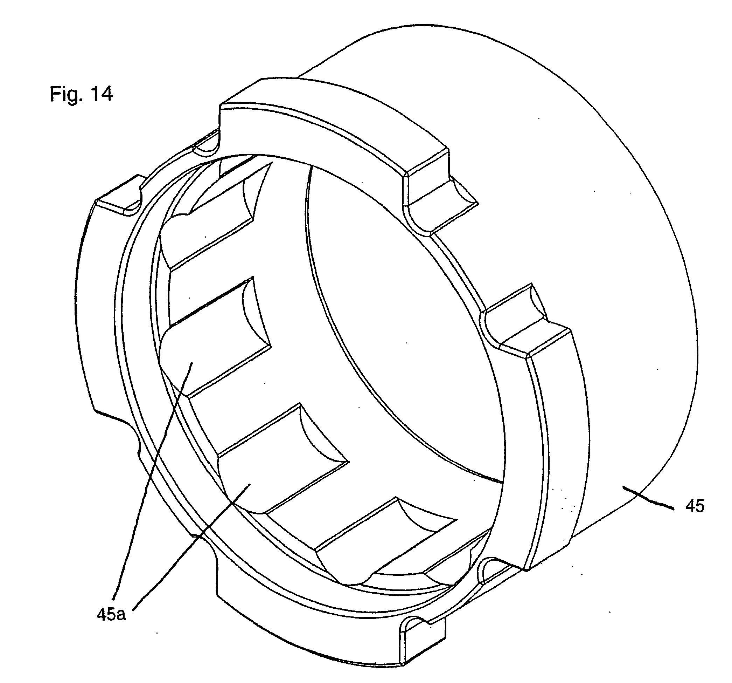

Die Ratsche 11 kann so ausgebildet sein wie in den Figuren 14 und 15 der Patentanmeldung PCT/CH2007/000243 beschrieben.

Während eines Ausschüttvorganges, welcher durch Drücken auf den Druckknopf 15 ausgelöst wird, wird die Anzeigetrommel 4 wieder in Gegenrichtung gedreht und verschiebt sich durch den Gewindeeingriff mit dem Innengewinde 12c der Injektionsvorrichtung wieder zurück in proximale Richtung (in Fig. 1 nach rechts). Dabei kann es auch zu einem in Umfangsrichtung wirkenden Anschlag der Anzeigetrommel 4 an das Gehäuse 12a, 12b der Injektionsvorrichtung und insbesondere an dem Gehäuseteil 12b kommen. Dieser Vorgang kann bei einer ungebremsten Ausschüttbewegung, bei welcher die Gewindestange 2 ohne Gegenkraft in distale Richtung bewegt wird, z.B. wenn kein Produktbehältnis eingelegt ist, zu einer starken Belastung und im Extremfall zu einer Verformung oder sogar Beschädigung der Anzeigetrommel 4 oder des Gegenstücks 12b führen. Es ist daher eine auf die Antriebsbewegung wirkende Bremseinrichtung 17, 18 vorgesehen, die weiter unten beschrieben wird.

Die Kupplung K1, die aus dem als Arretierhülse 14 ausgestalteten Kupplungsglied und der Kupplungshülse 5 gebildet wird, dient dazu, in bestimmten Schaltzuständen die Kupplungshülse 5 drehfest mit dem Gehäuse 12 zu koppeln bzw. für eine Drehung relativ zu dem Gehäuse 12 zu entkoppeln. Die Kupplung K1 ist bevorzugt entkoppelt bei einem Austausch des Produktbehältnisses 27, um das Abtriebsglied 2 wieder in proximale Richtung zurückschieben bzw. schrauben zu können, und bei einer Produktausschüttung, um das Abtriebsglied 2 in distale Richtung schrauben zu können. Die Kupplung K1 ist bevorzugt eingekuppelt, wenn das Produktbehältnis an der Antriebseinheit befestigt ist und das Betätigungselement 15 unbetätigt ist. Die Kupplung K1 wird gebildet durch Zähne auf der Außenseite der Kupplungshülse 5, welche in Zähne auf der Innenseite der Arretierhülse 14 eingreifen. Hierdurch wird die Kupplungshülse 5 relativ zur Arretierhülse 14 verdrehgesichert. Die Arretierhülse 14 ist verdrehgesichert und axial verschiebbar in der Injektionsvorrichtung, insbesondere relativ zu dem Gehäuse 12 und der Kupplungshülse 5 gelagert.

Während eines Ausschüttvorganges wird die Gewindehülse 13 durch Betätigung des Betätigungselements 15 in distale Richtung verschoben. Dabei drückt die Gewindehülse 13 auf das Lager 29, das in diesem Beispiel als Kugellager ausgebildet ist, jedoch auch als einfaches Gleitlager ausgebildet sein kann, wobei das Lager 29 auf die Arretierhülse 14 drückt, diese somit für einen Ausschüttvorgang in distale Richtung verschiebt und während eines Ausschüttvorgangs in distaler Position hält. Das Kupplungsglied 14 befindet sich somit distal der Abragungen der Kupplungshülse 5 für die Kupplung K1. Dadurch ist die Kupplung K1 für die Dauer des Ausschüttvorganges ausgekuppelt.

Beim Betätigen des Betätigungselements 15 verhalten sich die Kupplungen K1, K2 und K3 folgendermaßen: Durch Drücken des auf dem Kupplungsglied 10 und/oder Antriebswelle 7 sitzenden Druckknopfs 15 werden das Kupplungsglied 10 zusammen mit dem Druckknopf 15 und die Antriebswelle 7 in distale Richtung verschoben. Hierdurch kuppelt die Kupplung K2 ein, so dass die Antriebswelle 7 mit der Kupplungshülse 5 verdrehgesichert wird. Anschließend kuppelt die Kupplung K1 durch Verschiebung der Arretierhülse 14, auf welche die mit der Antriebswelle 7 verbundene Gewindehülse 13 über das axial verschiebbare Lager 29 drückt, aus. Die Kupplungen K1 und K2 können alternativ auch in umgekehrter Reihenfolge geschaltet werden.

Nach dem Einkuppeln von K2 und dem Auskuppeln von K1 kuppelt auch die Kupplung K3 durch Verschieben des Kupplungsglieds 10 relativ zum Dosierknopf 9 aus. Das Kupplungsglied 10, welches mit der Antriebswelle 7 verbunden ist, kann sich nach dem Auskuppeln der Kupplung K3 relativ zu dem Gehäuse 12 drehen. Die in der Antriebsfeder 3 während des Aufdosierens gespeicherte Energie oder Kraft kann auf die Antriebswelle 7 übertragen werden. Somit liegt an der Antriebswelle 7 ein Drehmoment an, welches mittels der eingekuppelten Kupplung K2 auf die Kupplungshülse 5 übertragen wird, welche sich zusammen mit der Antriebswelle 7 dreht und diese Drehbewegung auf das verdrehsicher mit der Kupplungshülse 5 gekoppelte Abtriebsglied 2 überträgt. Das in diesem Beispiel als Gewindestange ausgestaltete Abtriebsglied 2 setzt die Drehbewegung aufgrund des Gewindeeingriffs 2a, 6a mit dem Eingriffsglied 6, 26 in eine axiale Bewegung in distale Richtung um, so dass der am distalen Ende der Gewindestange 2 vorgesehene Flansch 1, der ebenfalls zum Abtriebsglied zugerechnet werden kann, in distale Richtung der Injektionsvorrichtung bewegt wird.

Da sich bei der Produktausschüttung die Gewindehülse 13 in entgegen gesetzte Richtung wie beim Aufdosieren dreht, dreht sich die Anzeigetrommel 4 ebenfalls in entgegen gesetzte Richtung wie beim Aufdosieren.

Im Normalfall, d.h. in dem Fall, bei dem die voreingestellte Produktdosis vollständig ausgeschüttet wird, verläuft der Ausschüttvorgang und insbesondere die Verschiebung des Abtriebsglieds 2 in distale Richtung so lange, bis der oben erwähnte in Umfangsrichtung wirkende Anschlag der Anzeigetrommel 4 anschlägt. Dies geschieht bevorzugt dann, wenn sich der durch das Fenster 12d ablesbare Wert auf 0 heruntergedreht hat.

In dem Fall, in dem der Benutzer der Vorrichtung das Beätigungselement 15 während der Produktausschüttung loslässt, kuppeln die Kupplungen in umgekehrter Reihenfolge, wie sie bei der Betätigung aus- bzw. eingekuppelt haben. Die Produktausschüttung wird unterbrochen, wobei durch das Fenster 12d der Wert ablesbar ist, der noch auszuschütten wäre, dass die voreingestellte Dosis komplett ausgeschüttet sein würde. Die Produktausschüttung kann dadurch fortgesetzt werden, dass das Betätigungselement 15 erneut gedrückt wird, wobei die Ausschüttung durch Loslassen des Betätigungselements 15 wieder gestoppt werden kann oder gewartet werden kann, bis die Produktausschüttung vollständig erfolgt ist.

Für den Fall, dass sich im Produktbehältnis weniger Produkt befindet als maximale Dosis auf der Anzeigetrommel angegeben ist, weist die in diesem Beispiel gezeigte Injektionsvorrichtung eine zusätzliche Einrichtung zur Begrenzung der letztmalig einstellbaren maximalen Dosis auf, um zu verhindern, dass eine größere Produktdosis eingestellt werden kann als sich Produkt in dem Produktbehältnis befindet. Hierzu ist ein Läufer 30 vorgesehen, der die Kupplungshülse 5 zumindest teilweise umgreift und in solch einem Eingriff mit der Kupplungshülse 5 ist, dass der Läufer 30 relativ zu der Kupplungshülse 5 drehfest und axial verschiebbar ist. Der Läufer 30 greift ferner mit einem an seinem äußeren Umfang gebildeten Gewinde in ein Innengewinde der Gewindehülse 13 ein. Diese Anordnung bewirkt, dass bei einer relativen Drehung zwischen Gewindehülse 13 und Kupplungshülse 5 eine Axialbewegung des Läufers 30 stattfindet, wobei bei keiner relativen Drehung der Läufer 30 keine Axialbewegung ausführt. Beim Einstellen einer Produktdosis wird die Gewindehülse 13 relativ zu der Kupplungshülse 5 verdreht, so dass der Läufer 30 in proximale Richtung wandert. Beim Ausschütten hingegen, findet keine Relativbewegung zwischen Kupplungshülse 5 und Gewindehülse 13 aufgrund des Kupplungseingriffs der Kupplung K2 statt. Die Läufer führt dann keine Bewegung aus. Nach mehrmaligem Dosieren und Produktausschütten gerät der Läufer 30 in einen axialen Anschlag mit der Antriebswelle 7, wodurch eine weitere Dosiserhöhung nicht mehr möglich ist, auch dann nicht mehr, wenn die Anzeige 4, 12d eigentlich mehr zuließe.

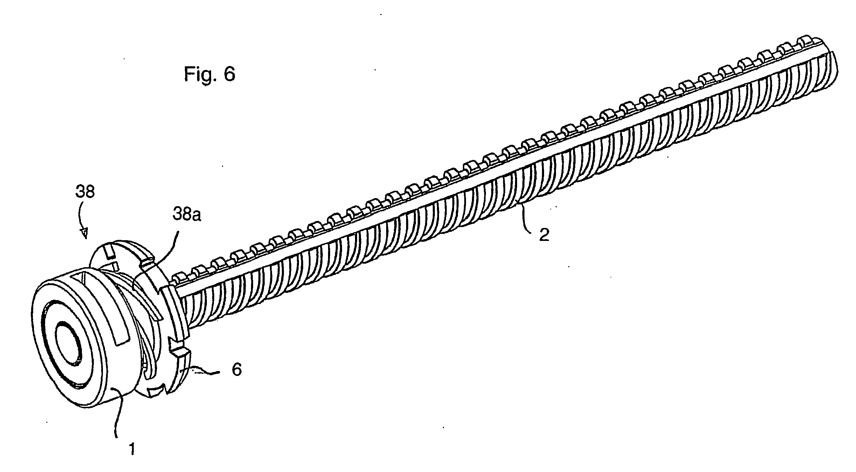

Der Verwender kann das Produktbehältnis 27 gegen ein neues austauschen. Hierzu entfernt er den Produktbehältnishalter 16 durch Drehung relativ zu dem Gehäuse 12 von der Antriebseinheit. Bei der Bewegung aus der befestigten Position in die unbefestigte Position des Produktbehältnisses 27, insbesondere beim Entriegeln des Bajonettverschlusses wird das Eingriffsglied 6, 26 zusammen mit dem Abtriebsglied 2 und der Kupplungshülse 5 relativ zu dem Gehäuse 12 und dem Kupplungsglied 14 in distale Richtung verschoben, wodurch die Kupplung K1 gelöst wird. Die für die Kupplung K1 vorgesehenen radial nach außen weisenden Abragungen der Kupplungshülse 5 befinden sich nun distal des Kupplungsglieds 14. Das Abtriebsglied 2 kann nun mit einer relativ geringen in proximale Richtung wirkenden Kraft in die Antriebseinheit geschraubt werden, da das Gewinde des Abtriebsglieds nicht selbsthemmend ist. Beim Zurückschrauben des Abtriebsglieds 2 wird die Kupplungshülse 5 relativ zu der Gewindehülse 13 und zwar entgegensetzt wie bei der Produktausschüttung verdreht, wodurch der Läufer 30 wieder axial verschoben wird in distale Richtung. Das Zurückschrauben kann zumindest über einen Teil des Gesamtwegs gegen die Kraft eines Federglieds erfolgen, das z.B. versucht, das Abtriebsglied in distale Richtung zu verschieben. Das Federglied kann z.B. zwischen dem Abtriebsglied 2 und der Antriebswelle 7 wirken bzw. angeordnet sein. Weitere vorteilhafte Federglieder werden insbesondere zu Figur 6 weiter unten beschrieben. Allgemein bevorzugt wird, dass die Kraft eines solchen Federglieds geringer ist als die für eine Wechselwirkung über den Kolben auf das Produkt von dem Abtriebsglied 2 erforderliche Kraft

Ferner wird beim Entfernen des Produktbehältnisses 27 das Halteglied 25, das dazu dient, das Produktbehältnis 27 in der Produktbehältnishalterung 16 zu fixieren, von der Feder 19 in distale Richtung verschoben bis es in einen Anschlag mit dem Eingriffsglied 6, 26 gerät. Dieser Anschlag verhindert, dass die Feder 19 sich bei entferntem Produktbehältnis 27 nicht vollständig entspannen kann. Dies ist vorteilhaft, da die Feder 19 auch bei einem entfernten Produktbehältnis 27 genügend Kraft aufbringen sollte, die Kupplung K3 in einem Kupplungseingriff zu halten.

Gemäß einem weiteren Aspekt kann ein gefederter Flansch realisiert werden, wie z.B. in Fig. 6 gezeigt.

Nach einem Wechsel des bevorzugt als Ampulle oder Karpulle ausgestalteten Produktbehältnisses 27 wird der Anwender, wie in der Bedienungsanleitung beschrieben, zum so genannten Entlüften aufgefordert. Dies ist erforderlich, da sich zum einen Luft in dem Produktbehältnis 27 befindet und zum anderen das Abtriebsglied 2 zuvor vollständig in die Antriebseinheit hineingeschoben wurde und durch den unterschiedlichen Füllstand des Produktbehältnisses 27 etwas Spiel zwischen dem Kolben 28 und dem Flansch 1 entstanden ist.

Figur 6 zeigt ein Abtriebsglied 2 mit einer seinem vorderen oder distalen Ende befestigten Flansch 1, der unverschiebbar mit der Gewindestange verbunden ist. Zwischen dem Flansch 1 und dem in Figur 6 gezeigten Gewindeeinsatz 6 ist ein Federglied 38 vorgesehen, welches z.B. durch schräg abstehende Federarme 38a realisiert werden kann. Diese Federarme 38a können am Flansch 1 oder/und am Gewindeeinsatz 6, befestigt sein. Ebenso könnte auch ein Elastomer am Flansch 1 oder/und am Gewindeeinsatz 6 angespritzt werden. Nach dem Einsetzen eines neuen Produktbehältnisses 27 kann es zu einem Spiel zwischen dem Flansch 1 und dem Kolben 28 kommen, was insbesondere auf unterschiedliche Füllstände bei vollen Produktbehältnissen 27 zurückzuführen ist, die eine gewisse Toleranz aufweisen.

Nach dem Einschieben des mit der Gewindestange 2 verbundenen Flansches 1 liegt der Flansch 1 gemäß der in Figur 1 gezeigten Ausführungsform unmittelbar an dem Gewindeeinsatz 6 an.

Gemäß der in Figur 6 gezeigten Ausführungsform wird der Flansch 1 jedoch durch das mindestens eine Federglied 38 vom Gewindeeinsatz 6 um eine vorgegebene Distanz in distale Richtung weggedrückt. Dies ermöglicht es, dass bei einem eingesetzten Produktbehältnis 27 oder beim Einsetzen des Produktbehältnisses 27 der Flansch 1 immer auf der proximalen Seite des Kolbens 28 zum Anliegen kommt, selbst wenn der Kolben 28 bei verschiedenen Produktbehältnissen bedingt durch Fertigungstoleranzen unterschiedlich weit in das Produktbehältnis 27 eingeschoben ist.

Herkömmliche Maßnahmen zur Beseitigung des Spiels zwischen Flansch 1 und Kolben 28 sind daher nicht mehr zwingend erforderlich und können z.B. sogar entfallen.

Wie aus Figur 1 zu erkennen ist, weist die Injektionsvorrichtung, insbesondere die Antriebseinheit eine Bremse 17, 18 auf, die ein sich drehendes Teil, in diesem Beispiel das Übertragungselement 7, K2, 5 oder/und die Antriebsbewegung abbremst. Bei herkömmlichen Injektionsvorrichtungen besteht bei Fehlanwendungen, z.B. dann, wenn kein Produktbehältnis eingelegt ist und dennoch eine Betätigung der Injektinosvorrichtung vorgenommen wird, die Gefahr von Überbelastung oder sogar einer Schädigung der Bauteile der Injektionsvorrichtung. Bei einem eingelegten Produktbehältnis 27 wird durch die Viskosität des Produkts bei der Produktausschüttung eine Dämpfung der auftretenden Kräfte und Bewegungen vorgenommen. Bei einem fehlenden Produktbehältnis fehlt ein solches Dämpfungsglied. Abhilfe schafft die Bremse 17, 18, die Überbelastungen vermeidet.

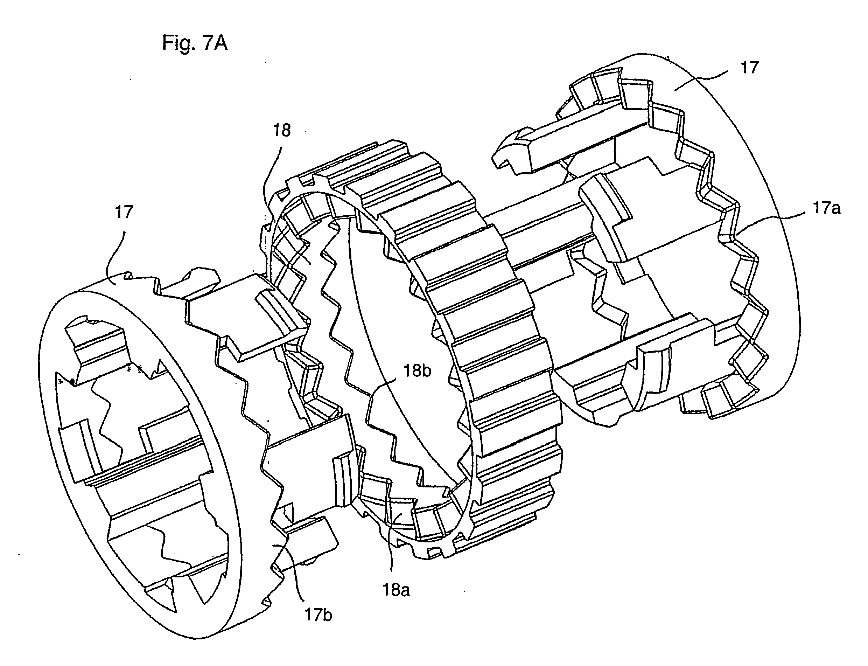

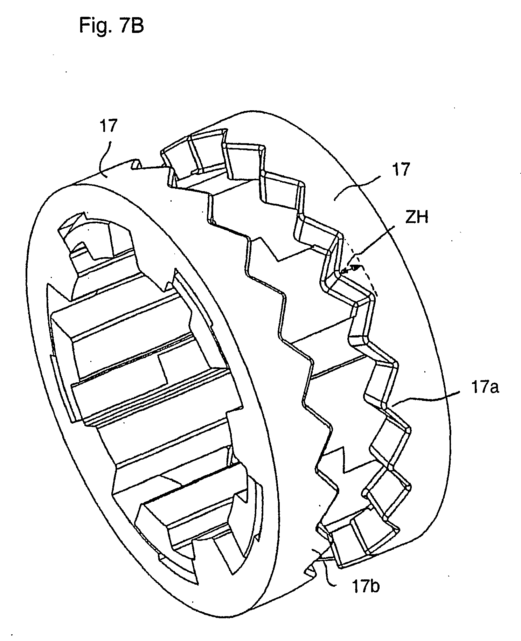

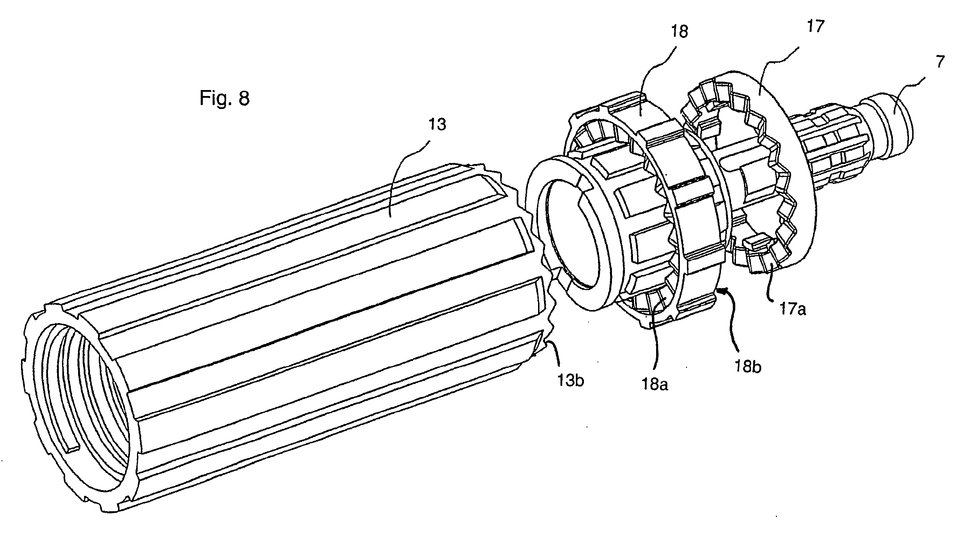

Die Figuren 7A, 7B und 8 zeigen in vergrößerter Darstellung einen für die Vorrichtung aus Figur 1 geeigneten Bremsmechanismus gemäß einer ersten und zweiten Ausführungsform, deren Wirkungsweisen ähnlich sind. Die erste Ausführungsform gemäß Figuren 7A, 7B weist zwei miteinander dreh- und vorzugsweise auch axialfest miteinander verrastete Bremsbackenhälften 17 auf, die zueinander weisende Profileabschnitte aufweisen, zwischen denen ein Ringspalt gebildet ist, in dem eine Bremsscheibe 18 aufgenommen ist. Der Ringspalt weist eine definierte Breite auf, wobei die Bremsbackenhälften alternativ relativ zu einander axial verschiebbar sein könnten. Die Bremsbacke 17 könnte einteilig ausgebildet sein. Die Bremsscheibe 18 ist relativ zu dem Gehäuse 12 drehfest und axial bewegbar aufgenommen, was durch die profilierte, in eine profilierte innere Umfangsfläche des Gehäuseteils 12b eingreifende äußere Umfangsfläche der Bremsscheibe, bewirkt wird. Zumindest eine Bremsbackenhälfte 17 oder die ganze Bremsbacke ist zumindest drehfest an dem Antriebsstrang bzw. dem Übertragungselement angeordnet. Die hülsenförmige Bremsbacke 17 weist radial nach innen weisende Abragungen auf, die in ein entsprechendes Profil der Antriebshülse 7 eingreifen. Die Bremsscheibe 18 kann sich zwischen den Bremsbackenhälften 17 bewegen. Die Bremsscheibe 18 ist verdrehgesichert z.B. durch Führung in einer Nut und axial verschiebbar in der Injektionsvorrichtung bzw. dem Gehäuseteil 12b gelagert. Die Bremsscheibe 18 ist an der Ober- und Unterseite durch stirnseitig umlaufend in beide Richtungen vorstehende Zähne 18a, 18b mit gleicher oder unterschiedlicher Zahnhöhe ZH verzahnt und zwischen der Gewindehülse 13 und der Bremsbacke 17, vorzugsweise mit einem kleinen Spiel von etwa einer Zahngröße oder Zahnhöhe ZH oder größer, gelagert oder verschiebbar eingespannt, welche entsprechende Gegenverzahnungen 13b bzw. 17a vorzugsweise mit entsprechender oder gleicher Zahnhöhe ZH besitzen.

Die Bremsbacke 17 wird durch die drehmomentfeste Verbindung zwischen Übertragungelement 7, K2, 5 z.B. bei einem Ausschüttvorgang oder beim so genannten Leerschießen, d.h. bei nicht eingelegtem Produktbehältnis, in eine Drehung Relativ zur Bremsscheibe 18 versetzt. Dabei sorgt die Anordnung der Bremsbackenverzahnungen 17a, 17b dafür, dass die Bremsscheibe 18 axial zwischen der Gewindehülse 13 und der Bremsbacke 17 oszilliert. Dadurch geraten die distale Verzahnung 18a und proximale Verzahnung 18b der Bremsscheibe 18 abwechselnd mit der entsprechenden Gegenverzahnung 17b und 17a in Kontakt. Durch die dabei auftretende Reibung, die elastische Deformation und vor allem die oszillierende Masse tritt ein entsprechender Verlust auf, wodurch die maximale Winkelgeschwindigkeit ω der sich drehenden Teile 13 und 17 begrenzt wird.

Die in Figur 8 gezeigte Ausführungsform arbeitet auf dem gleichen Prinzip, mit dem Unterschied, dass eine der zwei Bremsbackenhälften bzw. deren stirnseites Zahnprofil durch das Übertragungselement oder die mit dem Übertragungselement drehfest verbundene Gewindehülse 13 gebildet wird. Es kann zwischen den Profilen 17a und 13b ein fest definierter Abstand vorgesehen sein oder aber ein variabler Abstand indem die Bremsbackenhälfte 17 relativ zu der Gewindehülse 13 axial verschiebbar ist. Durch die Feder 19 könnten die Profile 13b und 17a aufeinander zu gerückt werden, so dass sie in den Eingriff mit den Profilen 18a und 18b geraten.

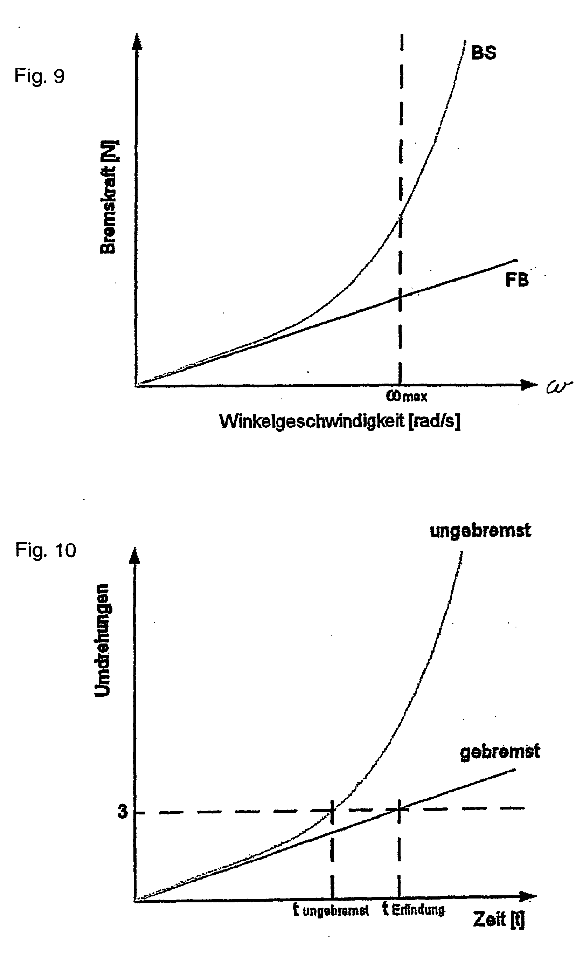

Durch die mit der Winkelgeschwindigkeit ω zunehmende Vibration oder Schwingung der Bremsscheibe 18 zwischen der Gewindehülse 13 und der Bremsbacke 18 nimmt die Bremskraft bei zunehmender Winkelgeschwindigkeit ω überproportional zu, so dass der in Figur 9 schematisch gezeichnete Verlauf BS der Bremskraft realisiert werden kann.

Figur 9 zeigt schematisch den Verlauf der mit der erfindungsgemäßen Bremsvorrichtung erzielbaren Bremskraft, wobei gesehen werden kann, dass die Bremskraft zunehmend mit der Winkel- oder Drehgeschwindigkeit ω ansteigt. Idealerweise ist die Bremskraft bis zur maximal zulässigen Winkelgeschwindigkeit ωₘₐₓ relativ gering oder Null und steigt bei der maximal zulässigen Winkelgeschwindigkeit ωₘₐₓ stark an.

Figur 10 zeigt den über die Zeit aufgetragenen Drehwinkel der Anzeigetrommel 4, welche im Ausführungsbeispiel drei vollständige Umdrehungen (3x360°) durchlaufen kann. Dabei kann aus Figur 10 gesehen werden, dass die Anzeigetrommel 4 drei vollständige Umdrehungen nach der Zeit t_{ungebremst} durchlaufen hat, welche kürzer ist, als die Zeit t_{Erfindung} im Falle einer gebremsten Drehbewegung der Anzeigetrommel 4, bei welcher sich der Drehwinkel im Idealfall linear in Abhängigkeit von der Zeit erhöht.

Durch die mittels der oszillierenden Bremsscheibe 18 erzeugten Bremskraft kann die maximal mögliche Winkelgeschwindigkeit ωₘₐₓ einer Ausschüttbewegung verringert oder begrenzt werden, so dass die sich zurückdrehende Anzeigetrommel 4 nur mit einer durch die Bremse 13, 17, 18 vorgegebenen maximalen Geschwindigkeit an in Umfangsrichtung wirkenden Anschlag oder das Gehäuseteil 12b anschlagen kann. Ist die Bremse 13, 17, 18 geeignet ausgelegt, ist die maximal mögliche Anschlaggeschwindigkeit der Anzeigetrommel 4 so gering, dass durch den Anschlagimpuls keine nennenswerten Verformungen oder Beschädigungen auftreten können. Alternativ zur Ausbildung der Bremse durch eine zwischen der Gewindehülse 13 und der Bremsbacke 17 oszillierende Bremsscheibe 18 können auch andere Bremsmechanismen verwendet werden.

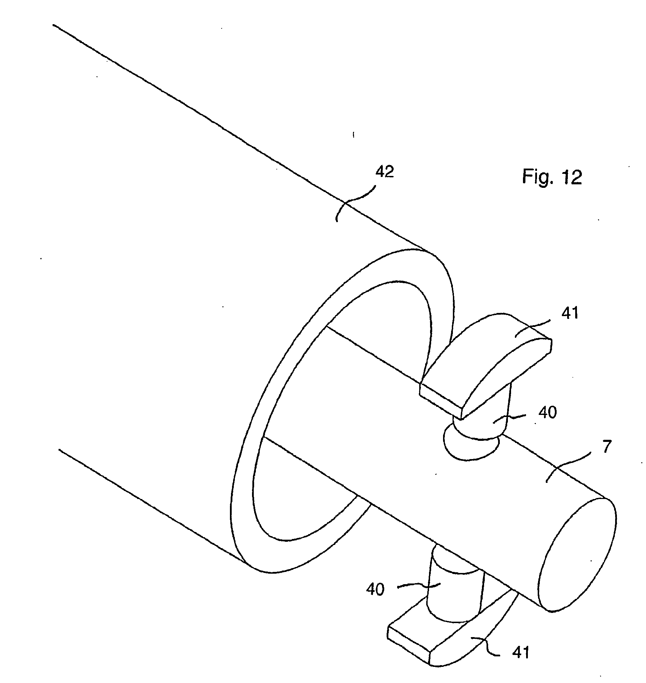

Beispielsweise kann die Bremse alternativ ergänzend oder auch gemäß einer dritten Ausführungsform durch eine in Figur 12 gezeigte Fliehkraftbremse realisiert werden, wobei z.B. an dem Übertragungselement 7, K2, 5 oder/und der Antriebswelle 7 und/oder einem anderen sich mit der Antriebswelle 7 drehenden Teil, wie z.B. dem Kupplungsglied 10, der Gewindehülse 13 oder der Anzeigetrommel 4, z.B. nach außen bewegbare Bremsbacken 41 angebracht sind, die eine Masse aufweisen und die die Drehung des rotierenden Teils mitmachen. Die Bremsbacken 41 können aber müssen nicht mittels einer Feder nach innen oder nach außen vorgespannt sein. Die Bremsbacken können durch die Fliehkraft verschwenkbar oder radial nach außen bewegbar sein, um in einen Bremseingriff mit einer Hülse 42, wie z.B. dem Gehäuse 12, kommen zu können. In der dritten Ausführungsform sind radial nach außen ragende Stifte 40 oder Befestigungen angebracht, an deren Enden sich z.B. mit der Feder vorgespannte Bremsbacken 41 befinden. Ist die Drehgeschwindigkeit des sich ungebremst oder nur teilweise gebremst drehenden Elements 4, 7, 10 und/oder 13 ausreichend groß, werden die Bremsbacken 41 durch die Zentrifugalkraft, ggf. auch zusätzlich durch Federunterstützung, radial nach außen bewegt und können z.B. an einer äußeren stehenden Hülse 42 in Anlage kommen und durch eine Reibung für die gewünschte Bremswirkung sorgen. Die äußere stehende Hülse kann z.B. auch durch das Gehäuse 12 oder das Gehäuseteil 12b gebildet werden.

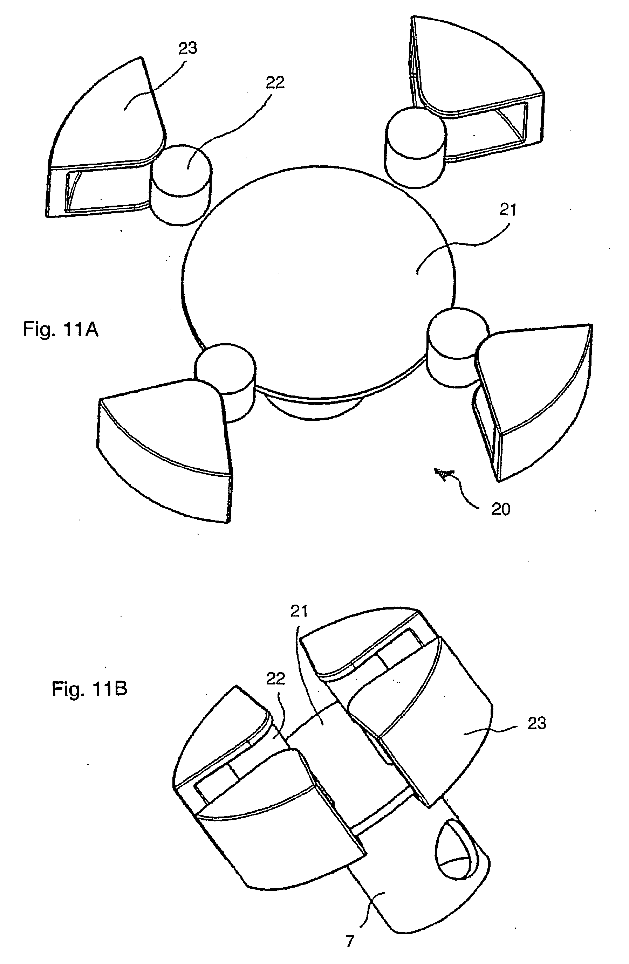

Bei einer vierten, in den Figuren 11A und 11B gezeigten Ausführungsform kann die Bremse als Wirbelstrombremse 20 ausgebildet werden, wobei eine Bremsscheibe 21 mit einem sich drehenden und zu bremsenden Teil, wie z.B. dem Übertragungselement 7, K2, 5, der Antriebswelle 7, der Gewindehülse 13 oder der Anzeigetrommel 4, und die mit der Bremsscheibe wechselwirkenden Elemente mit dem Gehäuse oder einem gehäusefesten Element oder mit einem Element relativ zu dem sich die Bremsscheibe dreht, verbunden sein können

Die Bremsscheibe 21 ist vorzugsweise aus einem sehr guten elektrischen Leiter gefertigt, wie z.B. Reinaluminium oder Kupfer. Als Material für die axial magnetisierten Magnete 22 können Seltenerdelegierungen, wie z.B. Neodym, verwendet werden. Das Permanentmagnetfeld kann mittels eines Magnetjochs 23 aus Eisen zum Luftspalt gelenkt werden, wo es möglichst senkrecht die Bremsscheibe 21 durchdringt. Die Bremskraft wird durch die Fläche und die Flussdichte im Luftspalt und den indizierte Strom in der Bremsscheibe 21 beeinflusst, wobei die Fläche möglichst groß, der Luftspalt möglichst klein und die Scheibenstärke möglichst groß sein sollte. Das Bremsmoment entsteht über den gemittelten Radius (Wirkradius). Es können Bremsen mit mehreren Magnetsystemen, welche auf eine Scheibe 21 wirken, ausgelegt werden.

Zur Berechnung der Stromdichte, der Bremsleistung und damit des Bremsmoments einer Wirbelstrombremse werden gewöhnlich Näherungsrechnungen verwendet. Unter Vernachlässigung des Effekts durch den Luftspalt wird ein einheitlicher zylindrischer magnetischer Fluss angenommen und vorausgesetzt, dass der Poldurchmesser gegenüber dem Radius der Scheibe 21 genügend klein ist. Bei hohen Drehzahlen wird die Näherung ungenau, da unter anderem die Magnetfelder, welche durch die Wirbelströme verursacht werden, zu einer nicht vernachlässigbaren Rückwirkung und damit zu Unlinearität führen.

Die Magnete 22 und das Magnetjoch 23 sind vorzugsweise mit dem Gehäuse 12 der Injektionsvorrichtung oder dem Gehäuseteil 12b oder einem anderen sich nicht drehenden Teil verbunden, um die gewünschte Wirbelstrombremswirkung der Bremsscheibe 21 erzeugen zu können.

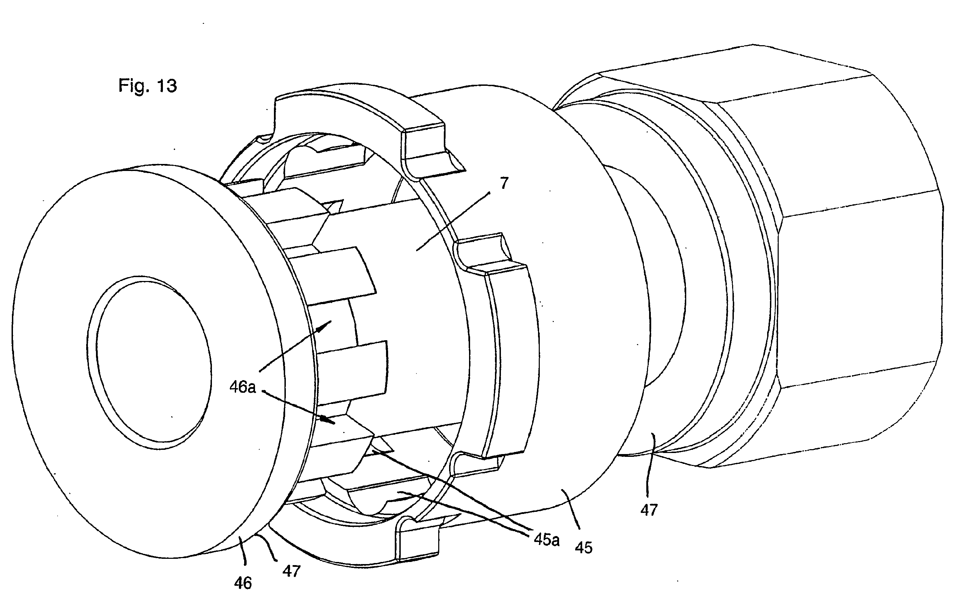

Gemäß einer vierten, in den Figuren 13 und 14 dargestellten Ausführungsform kann die Bremse als Fluidbremse ausgebildet werden. Wird ein normales Fluid als Bremsmedium verwendet, so wird die in Figur 4 für die Wirbelstrombremse gezeigte lineare Bremskennlinie FB erhalten. Soll jedoch eine in Abhängigkeit von der Winkelgeschwindigkeit ω stärker ansteigende Bremskraft realisiert werden, können so genannte nichtnewtonsche Fluide verwendet werden, bei welchen im Gegensatz zum newtonschen Fluid die Viskosität nicht konstant bleibt, sondern ansteigt, wenn sich eine auf das Fluid einwirkende Scherkraft erhöht, was bei zunehmender Geschwindigkeit der Fall ist. Es handelt sich dabei um so genannte anomalviskose Fluide.

Bei der Fluidbremse wird die Bremskraft über zwei sich gegeneinander bewegende Fluidoberflächen erzeugt. Insbesondere wird die Bremskraft über ein Fluidvolumen erzeugt, das durch eine Relativbewegung abgeschert wird. Die bei solchen Bewegungen auftretenden Scherspannungen entsprechen der Bremskraft. Das Volumen wird von einer zweigeteilten Kammer 45a, 46a gebildet, in der das Fluid angeordnet ist. Ein Kammerteil 46a ist in einem sich drehenden Teil 46 und die andere Kammer 45a in einem Teil 45 angeordnet relativ zu dem sich das Teil 46 drehen kann. Das Teil 46 kann z.B. mit der Antriebswelle7 oder dem Übertragungselement 7, K2, 5 oder einem anderen sich bei einer Produktausschüttung drehenden Teil drehfest verbunden sein. Das Teil 45 ist zumindest drehfest mit dem Gehäuse 12 oder einem gehäusefesten Teil verbunden. Zudem kann das Teil 45 relativ zu dem Gehäuse 12 axial bewegbar oder axialfest sein. Das hülsenförmige Teil 45 kann als Bremsengehäuse und das in der Hülse 45 gelagerte Teil 46 kann als Bremsenwelle bezeichnet werden. Im zusammengesetzten Zustand der Bremse sind die über den Außenumfang der Bremsenwelle verteilten Fluidkammerhälften 46a axial auf Höhe der über den Innenumfang des Bremsengehäuses verteilten Fluidkammerhälften 45a. Es können mehr, gleich viele oder weniger Fluidkammerhälften 45a als 46a vorgesehen sein. Zwischen dem Innendurchmesser des Bremsengehäuses 45 und dem Außendurchmesser der Bremsenwelle 46 jeweils im Bereich der Fluidkammerhälften 45a, 46a, ist im zusammengesetzten Zustand ein dünner Spalt, der so bemessen sein kann, dass Fluid in den Spalt transportiert wird oder dass kein oder so gut wie kein Fluid in den Spalt transportiert wird, wenn sich die Bremsenwelle 46 relativ zu dem Bremsengehäuse 45 dreht. Axial kann das Bremsengehäuse 45 auf beiden Stirnseiten mit gleitfähigen Dichtelementen 47 abgedichtet sein, so dass kein Fluid aus der Bremse austreten kann. Die Dichtelemente 47 können von einem Deckel gebildet werden. Als Deckel kann z.B. ein separates Teil vorgesehen sein oder die Kupplungswelle dienen.

**Bezugszeichenliste:**

| | | | |
|---|---|---|---|
| 1 | Flansch | 23 | Magnetjoch |
| 2 | Abtriebsglied / Kolbenstange | 24 | Stützring |
| 3 | Antriebsfeder / Spiralfeder | 25 | Halteglied |
| 4 | Anzeigetrommel | 26 | Eingriffsglied / Führungshülse |
| 5 | Kupplungshülse | 27 | Produktbehältnis |
| 6 | Eingriffsglied / Gewindeeinsatz | 28 | Kolben |
| 7 | Antriebsglied / Antriebswelle | 29 | Lager / Kugellager |
| 8 | Federhülse | 30 | Läufer |
| 9 | Dosierelement / Dosierknopf | 31 | Kappe |
| 10 | Kupplungselement | 38 | Federglied |
| 11 | Ratschenfeder | 40 | Radialführung |
| 12 | Gehäuse | 41 | Bremsbacke |
| 13 | Übertragungsglied / Gewindehülse | 42 | Bremshülse |
| 14 | Kupplungselement / Arretierhülse | 45 | Bremsengehäuse |
| 15 | Betätigungselement / Druckknopf | 46 | Bremsenwelle |
| 16 | Produktbehältnisaufnahme | 47 | Dichtelement |
| 17 | erstes Bremselement | 50 | Bajonetthülse |
| 18 | zweites Bremselement | K1 | erste Kupplung |
| 19 | Federelement / Wendelfeder | K2 | zweite Kupplung |
| 20 | Wirbelstrombremse | K3 | dritte Kupplung |
| 21 | Bremsscheibe | | |

## Patentansprüche

1. Injektionsvorrichtung zur Ausschüttung eines Produktes, umfassend
ein in die Injektionsvorrichtung eingesetztes Produktbehältnis (27),
ein Abtriebsglied (2), welches für eine Produktausschüttung auf das auszuschüttende Produkt, insbesondere über einen im Produktbehältnis (27) verschiebbar aufgenommenen Kolben (28) wirkt,
ein sich für einen Ausschüttvorgang bewegbares Element (14),
ein vom Verwender der Injektionsvorrichtung betätigbares, insbesondere axial verschlebbares Betätigungselement (15), wobei bei einer Betätigung des Betätigungselements (15) das Element (14) axial verschoben wird, so dass das Abtriebsglied (2) für eine Bewegung in oder entgegen einer Ausschüttrichtung entsperrt ist und
ein Federelement (19), welches das Produktbehältnis (27) für einen festen Sitz in einen Produktbehältnishalker (16) drückt,
**dadurch gekennzeichnet, dass**
das Federelement (19) gegen das bewegbare Element (14) drückt, um das Element (14) nach dem Loslassen des Betätigungselements wieder in eine Ausgangsposition zurück zu bewegen, so dass das Abtriebsglled (2) für eine Bewegung in oder entgegen einer Ausschüttrichtung gesperrt ist.

2. Injektionsvorrichtung nach dem vorhergehenden Anspruch, wobei das Federelement (19) zwischen dem Element (14) und dem Produktbehältnis (27) angeordnet ist.

3. Injektionsvorrichtung nach einem der vorhergehenden Ansprüche, wobei das Federelement (19) ein separates Teil oder ein von dem Element (14) gebildetes Teil ist.

4. Injektionsvorrichtung nach einem der vorhergehenden Ansprüche, wobei das Federelement (19) unmittelbar oder über ein Halteglied (25) auf das Produktbehältnis (27) wirkt.

5. Injektionsvorrichtung nach einem der vorhergehenden Ansprüche, wobei das Federelement (19) oder das Halteglied (25) einen Anschlag aufweist, der bei einem nicht eingelegten Produktbehältnis (27) eine vollständige Entspannung des Federelements (19) verhindert.

6. Injektionsvorrichtung nach einem der vorhergehenden Ansprüche, wobei das Element (14) Teil einer Kupplung (K1) ist und für einen Ausschüttvorgang aus dem Kupplungseingriff bewegt ist.

7. Injektionsvorrichtung nach einem der vorhergehenden Ansprüche, wobei das Element (14) relativ zu einem Gehäuse (12) axial verschiebbar und drehfest ist.

8. Injektionsvorrichtung nach einem der vorhergehenden Ansprüche, wobei das Element (14) so mit dem Abtriebsglied (2) gekoppelt ist, dass es eine Ausschüttbewegung des Abtriebsglieds (2) in einen unbefestigten Zustand der Injektionsvorrichtung sperrt.

9. Injektionsvorrichtung nach einem der vorhergehenden Ansprüche, wobei die Ausschüttbewegung des Abtriebsglieds (2) eine Drehbewegung ist.

10. Injektionsvorrichtung nach einem der vorhergehenden Ansprüche, wobei ein Eingriffgslied (6, 26) vorgesehen ist, welches in einem Eingriff mit dem Abtriebsglied (2) und zwischen einer ersten Position und einer zweiten Position bewegbar ist, wobei in der ersten Position das Abtriebsglied (2) von dem Element (14) entkoppelt ist, so dass das Abtriebsglied (2) in oder gegen eine Ausschüttrichtung bewegbar ist.

11. Injektionsvorrichtung nach einem der vorhergehenden Ansprüche, wobei das Eingriffsglied (26) so mit einer Befestigungseinrichtung gekoppelt ist, dass es beim Befestigen oder Lösen des Produktbehältnisses (27) an oder von der Antriebseinrichtung axial bewegt wird.

12. Injektionsvorrichtung nach einem der vorhergehenden Ansprüche, wobei das Betätigungselement (15) von dem Federelement (19) rücksetzbar ist.

## Claims

1. An injection device for dispensing a product, comprising
a product container (27) fitted into the injection device,
a drive output member (2) which acts on the product to be dispensed to dispense the product in particular by way of a plunger (28) which is displaceably accommodated in the product container (27)
an element (14) movable for a dispensing operation,
a moveable element which is moved for a dispensing operation,
an actuating element (15) which can be actuated by the user of the injection device and which in particular is axially displaceable, wherein upon actuation of the actuating element (15) the element (14) is displaced axially so that the drive output element (2) is released for a movement in or opposite relationship to a dispensing direction,
a spring element (19) which pushes the product container (27) for a firm seat into a product container holder (16),
**characterised in that** the spring element (19) presses against the movable element (14) to move the element (14) back into a starting position again after release of the actuating element so that the drive output member (2) is blocked for a movement in or in opposite relationship to a dispensing direction.

2. An injection device according to the preceding claim wherein the spring element (19) is disposed between the element (14) and the product container (27).

3. An injection device according to one of the preceding claims wherein the spring element (19) is a separate part or a part formed by the element (14).

4. An injection device according to one of the preceding claims wherein the spring element (19) acts on the product container (27) directly or by way of a holding member (25).

5. An injection device according to one of the preceding claims wherein the spring element (19) or the holding member (25) comprises a stop which prevents the spring element (19) from completely relaxing when no product container (27) is inserted.

6. An injection device according to one of the preceding claims wherein the element (14) is part of a coupling (K1) and is moved out of the coupling engagement for a dispensing operation.

7. An injection device according to one of the preceding claims wherein the element (14) is axially displaceable but non-rotatable relative to a housing (12).

8. An injection device according to one of the preceding claims wherein the element (14) is coupled to the drive output member (2) so that it blocks a dispensing movement of the drive output member (2) when the injection device is in a non-fixed condition.

9. An injection device according to one of the preceding claims wherein the dispensing movement of the drive output member (2) is a rotary movement.

10. An injection device according to one of the preceding claims wherein there is provided an engagement member (6, 26) which is movable in an engagement with the drive output member (2) and between a first position and a second position, wherein the drive output member (2) is uncoupled from the element (14) in the first position so that the drive output member (2) is movable in or in opposite relationship to a dispensing direction.

11. An injection device according to one of the preceding claims wherein the engagement member (26) is coupled to a fixing device so that it is moved axially when the product container (27) is fixed or released to or from the drive device.

12. An injection device according to one of the preceding claims wherein the actuating element (15) can be re-set by the spring element (19).

## Revendications

1. Dispositif d'injection pour l'administration d'un produit comprenant
un réservoir de produit (27) inséré dans le dispositif d'injection,
un organe d'entraînement (2) agissant, pour la distribution du produit, sur le produit à distribuer, plus particulièrement par l'intermédiaire d'un piston (28) logé de manière coulissant dans le réservoir de produit (27),
un élément (14) mobile pour un processus de distribution,
un élément d'actionnement (15) actionnable par l'utilisateur, plus particulièrement coulissant axialement, l'élément (14) étant déplacé axialement lors de l'actionnement de l'élément d'actionnement (15), de façon à ce que l'organe d'entraînement (2) soit débloqué pour un mouvement dans la direction de la distribution ou dans une direction opposée et
un élément de rappel élastique (19) qui presse le réservoir de produit (27) pour un appui ferme dans un support de réservoir de produit (16),
**caractérisé en ce que**
l'élément de rappel élastique (19) appuie contre l'élément mobile (14) pour déplacer l'élément (14) à nouveau vers une position de départ lors du relâchement de l'élément d'actionnement, de façon à ce que l'organe d'entraînement (2) soit bloqué pour un mouvement dans la direction de la distribution ou dans une direction opposée.

2. Dispositif d'injection selon la revendication précédente, l'élément de rappel élastique (19) étant disposé entre l'élément (14) et le réservoir de produit (27).

3. Dispositif d'injection selon l'une des revendications précédentes, l'élément de rappel élastique (19) étant un élément séparé ou une partie constitutive de l'élément (14).

4. Dispositif d'injection selon l'une des revendications précédentes, l'élément de rappel élastique (19) agissant directement ou par l'intermédiaire d'un organe de maintien (25) sur le réservoir de produit (27).

5. Dispositif d'injection selon l'une des revendications précédentes, l'élément de rappel élastique (19) ou l'organe de maintien (25) comprenant une butée qui empêche une détente complète de l'élément de rappel élastique (19) lorsque le réservoir de produit (27) n'est pas inséré.

6. Dispositif d'injection selon l'une des revendications précédentes, l'élément (14) étant une partie d'un accouplement (K1) et étant désaccouplé pour un processus de distribution.

7. Dispositif d'injection selon l'une des revendications précédentes, l'élément (14) étant mobile axialement et solidaire en rotation par rapport à un boîtier (12).

8. Dispositif d'injection selon l'une des revendications précédentes, l'élément (14) étant couplé avec l'organe d'entraînement (2) de façon à bloquer un mouvement de distribution de l'organe d'entraînement (2) dans un état non fixé du dispositif d'injection.

9. Dispositif d'injection selon l'une des revendications précédentes, le mouvement de distribution de l'organe d'entraînement (2) étant un mouvement de rotation.

10. Dispositif d'injection selon l'une des revendications précédentes, un organe d'engrènement (6, 26) étant prévu, qui est mobile en engrènement avec l'organe d'entraînement (2) et entre une première position et une deuxième position, l'organe d'entraînement (2) étant, dans la première position, désaccouplé de l'élément (14), de façon à ce que l'organe d'entraînement (2) soit mobile dans une direction de distribution ou dans une direction opposée.

11. Dispositif d'injection selon l'une des revendications précédentes, l'organe d'engrènement (26) étant couplé avec un dispositif de fixation de façon à ce qu'il soit déplacé axialement lors de la fixation ou du détachement du réservoir du produit (27) sur ou du dispositif d'entraînement.

12. Dispositif d'injection selon l'une des revendications précédentes, l'élément d'actionnement (15) pouvant être rappelé par l'élément de rappel élastique (19).
